(19) Europäisches Patentamt
European Patent Office
Office européen des brevets

(11) **EP 3 446 652 A1**

(12) **EUROPEAN PATENT APPLICATION**
published in accordance with Art. 153(4) EPC

(43) Date of publication:
**27.02.2019 Bulletin 2019/09**

(21) Application number: **16889792.4**

(22) Date of filing: **09.02.2016**

(51) Int Cl.:
***A61B 18/00*** (2006.01)

(86) International application number:
**PCT/JP2016/053803**

(87) International publication number:
**WO 2017/138090 (17.08.2017 Gazette 2017/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Murakumo Corporation Tokyo 153-0061 (JP)**

(72) Inventor: **TSUTSUMI, Yohei Tokyo 153-0061 (JP)**

(74) Representative: **Plougmann Vingtoft a/s Strandvejen 70 2900 Hellerup (DK)**

(54) **ULTRASONIC OSCILLATION DEVICE, AND METHOD AND PROGRAM RELATING THERETO**

(57) An object is to accurately grasp the position and the like of the transducer in an ultrasonic oscillator that retains the transducers in a deformable concave portion and moves the transducer in accordance with deformation of the concave portion. An ultrasonic oscillator retains transducers on a deformable concave portion, the transducers move in conformity with deforming of the concave portion, and the controller comprises receiver that receives sensing signals output from the sensing side transducers which sensed the ultrasonic waves oscillated from the oscillation side transducers, a distance calculation unit that calculates the distance between the oscillation side transducer and the sensing side transducer based on propagation time of the direct wave, an angle calculation unit that calculates a visual angle between a center axis of oscillation and the sensing side transducer viewed from the oscillation side transducer based on the sensing signal related to the direct wave, and a correction unit that corrects position information indicating a relative positional relationship between the plurality of transducers by using the distance and the visual angle.

*FIG. 7*

**Description**

TECHNICAL FIELD

The present invention relates to an ultrasonic oscillator.

BACKGROUND ART

In the prior art, there has been proposed an ultrasonic probe including: an oscillator for treatment that has a plurality of arrayed first transducers and applies ultrasonic waves for treatment to a subject; and an oscillator for diagnosis that has a plurality of arrayed second transducers and applies ultrasonic waves for diagnosis to the subject and receives the ultrasonic waves for diagnosis reflected by the subject, in which the oscillator for treatment and the oscillator for diagnosis are stacked on top of one another (see WO 2004/066856).

In addition, there has been proposed an ultrasonic treatment device including: an ultrasonic generation source that generates ultrasonic waves for treatment; and drive means that drives the ultrasonic generation source such that the frequencies of the ultrasonic waves for treatment generated from the ultrasonic generation source change with time (see Japanese Patent Application Laid-open No. H8-131454).

PRIOR ART DOCUMENT

PATENT LITERATURE

Patent Document 1: WO 2004/066856
Patent Document 2: Japanese Patent Application Laid-open No. H8-131454

SUMMARY OF THE INVENTION

PROBLEM TO BE SOLVED BY INVENTION

In the prior art, there have been proposed devices that oscillate ultrasonic waves to heat targets. For the conventional devices, however, users are required to mechanically control the positions of ultrasonic transducers with high accuracy so as to adjust correctly the positions that are heated using ultrasonic waves, and thus the burden that falls on the users operating such devices is heavy. Further, when such heating is used for treatment, persons to be treated are also forced to bear heavy burdens due to the considerable time required for preparations before the heating, severe limitations on the persons' body movement during the treatment, and so on.

In consideration of the above problem, the applicant of the present invention has invented an ultrasonic oscillator capable of accurately heating a target with a device configuration and a user operation simpler than before (PCT/JP2015/056750). The ultrasonic oscillator comprises a plurality of transducers that oscillates and/or senses the ultrasonic waves; a retention portion that retains the plurality of transducers on a deformable concave portion forming a concave thereof opposing the heating target, so as to retain the plurality of transducers movable in conformity with the concave portion deforming in a state where the plurality of transducers is arranged along the concave; and a controller that controls the transducers so as to oscillate the ultrasonic waves.

However, compared with the conventional apparatus in which the position and the like of the transducers are determined in advance with mechanical precision, a configuration in which the transducers are retained on a deformable concave portion and the transducers move in accordance with the deformation of the concave portion is disadvantageous in precision such as the position where the transducer is held. On the other hand, when attempting to hold the transducer at a predetermined precise position or the like, the device configuration becomes complicated/expensive, and advantages such as simple device configuration and user operation are lost.

In view of the above problems, it is an object of the present invention to accurately ascertain the positions and the like of transducers in an ultrasonic oscillator that retains the transducers on a deformable concave portion.

MEANS FOR SOLVING THE PROBLEM

In order to solve the above-mentioned problem, the present invention adopts the following means. That is, an example of the present disclosure is an ultrasonic oscillator that heats a target using ultrasonic waves, the ultrasonic oscillator comprising: a plurality of transducers that oscillates and/or senses the ultrasonic waves; a retention portion

2

that retains the plurality of transducers on a deformable concave portion forming a concave thereof opposing the heating target, so as to retain the plurality of transducers movable in conformity with the concave portion deforming in a state where the plurality of transducers is arranged along the concave; and a controller that controls the transducers so as to oscillate the ultrasonic waves, wherein the controller comprises: receiver that receives sensing signals output from the transducers which sensed the ultrasonic waves oscillated from any of the plurality of transducers, a direct wave specifying unit that specifies a sensing signal related to a direct wave included in the received sensing signals, a distance calculation unit that calculates the distance between the oscillation side transducer and the sensing side transducer based on time from oscillation to sensing of the direct wave, an angle calculation unit that calculates a visual angle between a center axis of oscillation and the sensing side transducer viewed from the oscillation side transducer, based on the sensing signal related to the direct wave, and a correction unit that corrects position information indicating a relative positional relationship between the plurality of transducers by using the distance and the visual angle.

EFFECTS OF INVENTION

According to the present invention, it is possible to accurately ascertain the positions and the like of the transducers in the ultrasonic oscillator that retains the transducers on the deformable concave portion.

BRIEF DESCRIPTION OF DRAWINGS

FIG. 1 is a diagram schematically showing the ultrasonic oscillator according to the embodiment,
FIG. 2 is a diagram schematically showing a membrane included in the ultrasonic oscillator according to the present embodiment,
FIG. 3 is a diagram schematically showing a functional configuration of the controller according to the present embodiment,
FIG. 4 is a flowchart showing the flow of the startup/operation process in the present embodiment,
FIG. 5 is a flowchart showing the flow of the process of determining the relative position/posture of the transducers and the membrane shape (calibration process) in the present embodiment,
FIG. 6 is a flowchart showing the flow of the relative position/posture determination process of the transducers according to the present embodiment,
FIG. 7 is a diagram showing the relationship between the orientation (posture) of the oscillation-side transducer and the sensing-side transducer and the direct wave oscillated from the oscillation-side transducer and sensed by the sensing-side transducer in the present embodiment,
FIG. 8 is a flowchart showing the flow of the membrane shape determination process in the present embodiment,
FIG. 9 is a diagram showing the relationship between the orientation (posture) of the oscillation-side transducer and the sensing-side transducer and the reflected wave oscillated from the oscillation-side transducer and sensed by the sensing-side transducer in the present embodiment,
FIG. 10 is a flowchart showing the flow of the scanning and imaging process in the present embodiment,
FIG. 11 is a schematic diagram showing calculation contents for focusing by phase control in the present embodiment, and
FIG. 12 is a flowchart showing the flow of the heating process in the present embodiment.

DESCRIPTION OF EMBODIMENTS

Hereinafter, a description will be given of an embodiment of an ultrasonic oscillator according to the present disclosure based on the drawings. However, the following embodiment is only for illustration purpose and does not limit the ultrasonic oscillator according to the present disclosure to the following specific configuration. For the implementation of the ultrasonic oscillator, the specific configuration corresponding to the embodiment is appropriately employed or various improvements or modifications may be made.

<System Configuration>

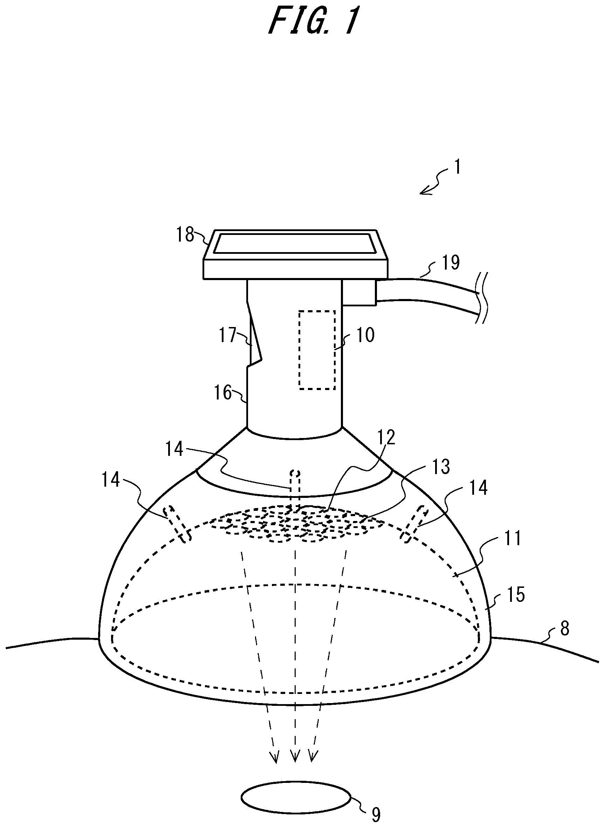

FIG. 1 is a diagram schematically showing the ultrasonic oscillator 1 according to the embodiment. The ultrasonic oscillator according to the embodiment aims to increase the efficiency of heat treatment for a target 9 in a living body 8 using ultrasonic waves and the status confirmation of the treatment to reduce a burden on a physician (user) and realize more accurate scanning and heating than ever before.
The ultrasonic oscillator 1 according to the embodiment has a membrane 11 having a substantially hemispherical shape, an ultrasonic transducer array 13 constituted by a plurality of ultrasonic transducers 12 provided on the membrane 11, an actuator 14 provided on the outer surface of the membrane 11, a housing 15 in which the membrane 11 and the like is provided, a handle 16 provided at the upper end of the housing 15 and handled by a user, a lever 17 by which the user is allowed to perform an operation while holding the handle 16, a controller 10 that controls the ultrasonic transducers 12, the actuator 14, and the like, a display 18 that is provided at the upper end of the handle 16 and displays image data or the like output from the controller 10 thereon, and a cable 19 connected to an external device. The cable 19 may include a communication cable, a power cable, a water-cooling pipe used to cool the ultrasonic transducers 12, or the like. However, it is possible to appropriately make the omission, replacement, and addition of the specific hardware configuration of the ultrasonic oscillator 1 according to the embodiment. For example, the display may not be included in the configuration of the ultrasonic oscillator, and a display connected to an outside in a wired or wireless fashion may be used instead. Likewise, the controller may be wired or wirelessly connected externally.

The user is allowed to hold the handle 16 and freely move the ultrasonic oscillator 1 on the front surface of a living body 8 including a heating target 9. In addition, the user is allowed to instruct the controller 10 to heat the heating target 9 (oscillate ultrasonic waves from the ultrasonic transducers 12) by operating the lever 17 based on the confirmation of a display content on the display 18 while holding the handle 16.

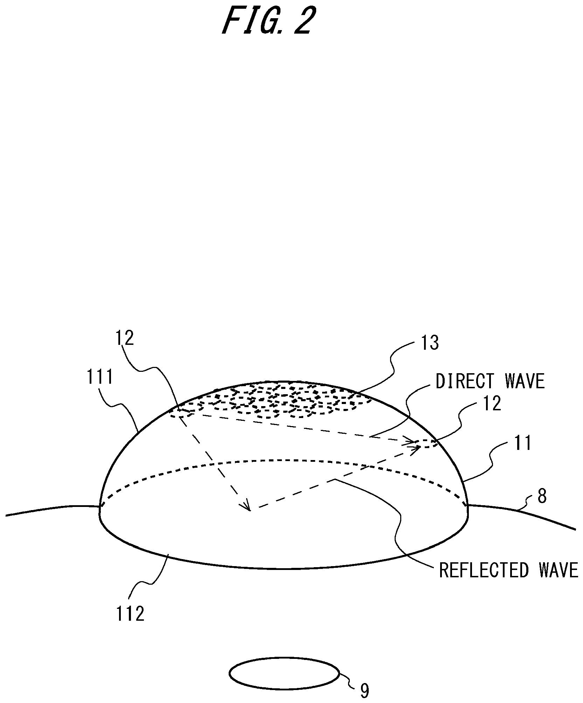

FIG. 2 is a diagram schematically showing a membrane 11 included in the ultrasonic oscillator 1 according to the present embodiment. The membrane 11 is a member having a substantially hemispherical shape as a whole, the membrane 11 having a concave portion 111 that forms a concave opposing the heating target 9 in a state in which the ultrasonic oscillator 1 is directed to the heating target 9 and having a contact portion 112 that seals the opening of the concave portion 111 and contacts the living body 8 including the heating target 9. The concave portion 111 has the substantially hemispherical shape in the embodiment, but it may employ other shapes. For example, the concave portion 111 may employ a cone shape, a truncated cone shape, a half-turn ellipsoid shape, shapes approximating these shapes, or the like.

The outer shell of the membrane 11 is made of an elastic material, and the inside of the membrane 11 is filled with gel. Therefore, the concave portion 111 and the contact portion 112 of the membrane 11 are made deformable. Note that the inside of the membrane 11 may be filled with any substance so long as the substance is adaptable to the deformation of the concave portion 111 and may be filled with, for example, liquid. In addition, for the propagation of ultrasonic waves, the membrane 11 and the substance filling the inside of the membrane 11 is preferably a medium that does not greatly absorb the ultrasonic waves, realizes a propagation speed close to a speed at which the ultrasonic waves pass through a living body 8, and has uniformity hardly causing irregularities.

Moreover, the plurality of ultrasonic transducers 12 is held side by side on the inner side surface of the concave portion 111 of the membrane 11. Thus, the plurality of ultrasonic transducers 12 is arranged along the concave opposing the heating target 9 to constitute the ultrasonic transducer array 13 having a concave shape. That is, in the embodiment, the membrane 11 also functions as a retention portion that retains the ultrasonic transducers 12. However, the plurality of ultrasonic transducers 12 may be retained on the outer side surface of the concave portion 111 of the membrane 11.

The actuator 14 (driver) is connected to the membrane 11 and deforms the concave portion 111 of the membrane 11 by driving. In the embodiment, a plurality of linear actuators 14 is provided so as to connect the membrane 11 and the housing 15 surrounding the membrane 11 to each other. When the respective linear actuators 14 implement drive according to an instruction from the controller 10, the concave portion 111 deforms. However, the configuration of the driver shown in the embodiment is just an example, and means for deforming the concave portion 111 with a driver is not limited to the example of the embodiment. For example, a mechanism similar to a mechanism for opening/closing an umbrella may be provided so as to surround the concave portion 111 of the membrane 11 and opened/closed with one actuator to deform the concave portion 111. In addition, the driver is allowed to deform the concave portion 111 even if it is not connected to the housing 15.

In the present embodiment, an example of deforming the concave portion by using the actuator is described, but other methods may be adopted as a method of deforming the concave portion. For example, a method of deforming the concave portion by adjusting the amount of gel inside the membrane may be adopted. In this case, the controller 10 can deform the concave portion by adjusting the gel amount in accordance with a map or a function, which is retained in advance, showing the correspondence relationship between the gel amount and the shape (for example, curvature) of the concave portion.

Moreover, in the embodiment, the plurality of ultrasonic transducers is also used as sensing means (sensors) for sensing the direct waves or reflected waves of ultrasonic waves oscillated from other ultrasonic transducers. When sensing the direct waves or reflected waves of the ultrasonic waves oscillated from other ultrasonic transducers, the plurality of ultrasonic transducers outputs sensing signals corresponding to the amplitudes, frequencies, phases, or the like of the sensed ultrasonic waves to the controller 10. After receiving the sensing signals, the controller 10 generates three-dimensional image data on a reflecting target including the heating target 9 using sensing signals based on reflected waves among the received sensing signals.

In the embodiment, the ultrasonic transducers 12 are also used as the sensors for sensing. However, the sensors for sensing may be provided separately from the transducers for oscillation. In addition, oscillation and sensing for heating and oscillation and sensing for imaging may be performed using the same ultrasonic transducer array or may be performed using an ultrasonic transducer array for heating and an ultrasonic transducer array for imaging that are separately provided. When the ultrasonic transducer array for heating and the ultrasonic transducer array for imaging are separately provided, they may be layered on the concave portion 111 or may be dispersedly arranged on the same layer.

The controller 10 is a computer having a central processing unit (CPU), a random access memory (RAM), a read only memory (ROM), a storage (auxiliary memory), a communication interface, or the like (each of which is omitted in the figures). The controller 10 performs the following various control processing in such a way that the CPU runs a program read from the storage and developed into the RAM or a program stored in the ROM. However, it is possible to appropriately make the omission, replacement, and addition of the specific hardware configuration of the controller 10 according to the embodiment. In addition, the controller 10 is not limited to a single unit. The controller 10 may be realized by a plurality of units using the technology of so-called cloud computing or distributed computing or the like.

The controller 10 controls the ultrasonic transducers 12 so as to oscillate ultrasonic waves. More specifically, in a state in which the focal point of ultrasonic waves to be oscillated from the plurality of ultrasonic transducers 12 is positioned at the heating target 9, the controller 10 controls the respective transducers 12 so as to oscillate the ultrasonic waves to heat the heating target 9. Here, the controller 10 controls the plurality of ultrasonic transducers 12 so as to adjust the phases of the ultrasonic waves oscillated from the ultrasonic transducers 12 to perform the control of a heating position of the ultrasonic waves, the cancellation of the heating, or the like. Note that as a specific method for changing a heating position (focal position) under phase control, it is possible to employ an ultrasonic phased array technology in which the phases of ultrasonic waves output from respective transducers 12 are made different to generate pseudo different oscillation points to control the direction of a combined wave. In the embodiment, the controller 10 controls the phases of the ultrasonic waves output from the respective transducers 12 so as to control the direction of a combined wave and adjust the heating position.

In addition, the controller 10 controls the actuator 14 so as to push/pull the concave portion 111 to be deformed and adjust the oscillation directions of the ultrasonic waves oscillated from the plurality of ultrasonic transducers 12 to control the heating position (focal position) of the ultrasonic waves oscillated from the plurality of ultrasonic transducers 12. As described above, the plurality of ultrasonic transducers 12 that oscillates the ultrasonic waves is provided on the concave portion 111 of the membrane 11 to constitute the ultrasonic transducer array 13. Therefore, when the concave portion 111 deforms by means of the actuators 14, the plurality of ultrasonic transducers 12 moves correspondingly. As a result, the oscillation directions of the ultrasonic waves from the respective ultrasonic transducers 12 change. The ultrasonic oscillator according to the embodiment deforms the concave portion 111 in the way described above, which makes it possible to adjust the oscillation directions of ultrasonic waves and a heating position.

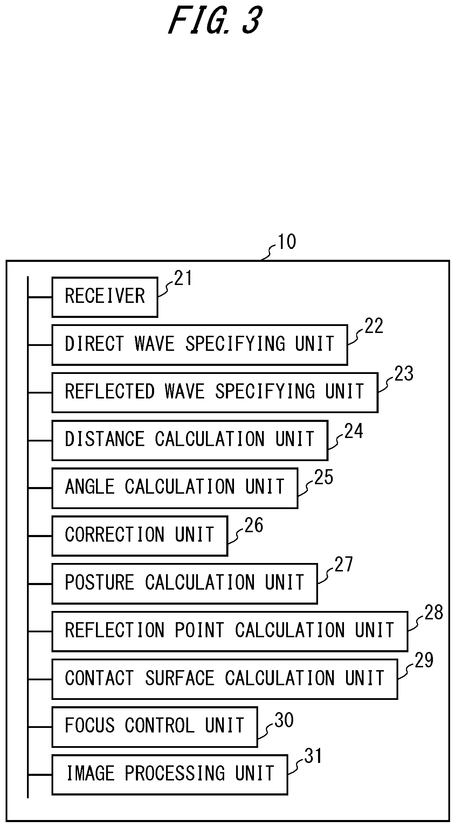

FIG. 3 is a diagram schematically showing a functional configuration of the controller 10 according to the present embodiment. The CPU executing the program read out from the storage and developed in the RAM and the program stored in the ROM so that so that the controller 10 functions as a receiver 21, a direct wave specifying unit 22, a reflected wave specifying unit 23, a distance calculation unit 24, an angle calculation unit 25, a correction unit 26, a posture calculation unit 27, a reflection point calculation unit 28, a contact surface calculation unit 29, a focus control unit 30, and an image processing unit 31.

The receiver 21 receives a sensing signal output from the ultrasonic transducer 12 which sensed the ultrasonic wave oscillated from another ultrasonic transducer 12.

The direct wave specifying unit 22 specifies a sensing signal related to a direct wave included in the received sensing signal.

The reflected wave specifying unit 23 specifies the sensing signal related to a reflected wave included in the received sensing signal.

The distance calculation unit 24 calculates the distance between the oscillation-side transducer and the sensing-side transducer based on the time from the oscillation to the sensing of the direct wave, and further calculate the path length of the reflected wave from the oscillation-side transducer to the sensing-side transducer based on the time from the oscillation to the sensing of the reflected wave. Here, the distance calculation unit 24 calculates the distance by multiplying the propagation speed by the time from the oscillation to the sensing.

The angle calculation unit 25 calculates a visual angle between the center axis of oscillation (the oscillation axis of main lobe) and the sensing-side transducer viewed from the oscillation-side transducer based on the sensing signal related to the direct wave. The angle calculation unit 25 further calculates a visual angle between the center axis of oscillation and the reflection point viewed from the oscillation-side transducer based on the sensing signal related to the reflected wave, and calculates the incident angle from the reflection point to the sensing-side transducer based on the intensity of the reflected wave. Here, the angle calculation unit 25 calculates the visual angle by calculating the oscillation angle having the spectrum corresponding to the spectrum of the sensing signal based on the correspondence relationship between the oscillation angle and the spectrum held in advance. The "visual angle" is an angle indicating how many degrees the point (the object such as another transducer or reflection point, etc.) deviates from the center axis of oscillation extending in the orientation of the transducer. For example, the visual angle between the central axis of the oscillation and the target viewed from the oscillation-side transducer is an angle formed by "the direction of the center axis passing through the oscillation plane of the oscillation-side transducer (the center direction in which the ultrasonic wave oscillated from the oscillation-side transducer is directed)" and "a line connecting the center of the oscillation plane of the oscillation-side transducer and the object". In other words, the "visual angle" is equal to the zenith angle when viewing the object in the spherical coordinate system selected with the center of the oscillation plane of the oscillation-side transducer as the origin and the central axis of the oscillation plane as the z axis. In addition, the angle calculation unit 25 calculates the incident angle by comparing the estimated intensity of the sensing signal when the sensing-side transducer senses vertically and the intensity of the actually received sensing signal.

The correction unit 26 corrects the position information indicating the relative positional relationship of the plurality of ultrasonic transducers 12 using the distance and the visual angle.

The posture calculation unit 27 calculates the orientation (posture) of the oscillation side transducer based on the visual angle between the center axis of oscillation and the first sensing side transducer viewed from the oscillation side transducer, the visual angle between the center axis of oscillation and the second sensing side transducer viewed from the oscillation side transducer, the position information of the oscillation side transducer, the position information of the first sensing side transducer, and the position information of the second sensing side transducer. In this case, information (visual angle and position information) related to the two sensing side transducers of the first sensing side transducer and the second sensing side transducer is used for calculating the posture. However, information (visual angle and position information) related to three or more sensing side transducers may be used for calculating the posture, and the number of sensing side transducers is not limited.

The reflection point calculation unit 28 calculates the reflection point position information indicating the position of the reflection point on the contact surface where the contact portion 112 contacts the living body 8, based on the position information of the ultrasonic transducers 12, the orientations of the ultrasonic transducers 12, the path length of the reflected wave, a visual angle between the center axis of oscillation and the reflection point viewed from the oscillation side transducer, and the incident angle.

The contact surface calculation unit 29 calculates contact surface shape information indicating the shape of the contact surface where the contact portion 112 contacts the living body 8 by using the plurality of the reflection point position information.

The focus control unit 30 controls the focal position of ultrasonic waves oscillated from the plurality of the ultrasonic transducers 12, by calculating time required for ultrasonic waves to reach the focal position from each ultrasonic transducer 12 via the contact surface based on the position information indicating the relative positional relationship between the plurality of ultrasonic transducers 12, orientations of the ultrasonic transducers 12, and the contact surface shape information, and adjusting phase (oscillation timing) of the ultrasonic waves by each of the plurality of the ultrasonic transducers 12 in accordance with the calculation result.

Further, focus control unit 30 controls the actuator 14 so as to deform the concave portion 111 and adjust oscillation directions of the ultrasonic waves oscillated from the plurality of ultrasonic transducers 12 based on the result of comparing position information indicating the relative positional relationship between the plurality of ultrasonic transducers 12 and orientations of the plurality of ultrasonic transducers 12 with aiming position and aiming orientation of the plurality of ultrasonic transducers 12, thereby controls the focal position of the ultrasonic waves oscillated from the plurality of ultrasonic transducers 12.

The image processing unit 31 generates and outputs image data on a reflecting target including the target 9 using sensing signals based on reflected waves among the received sensing signals (The format of the image data is not limited. For example, the image data may be either two-dimensional image data or three-dimensional image data). The image processing unit 31 compares the position of the target 9 with the focal position in the image data and generates and outputs instructing information for guiding the focal position to the position of the target 9.

<Control of Apparatus>

Next, control executed in the ultrasonic oscillator 1 according to the present embodiment will be described. It should be noted that the specific contents, order, etc. of the processes described in the present embodiment are examples for implementation. Specific processing contents, order and the like may be appropriately selected according to the mode of implementation.

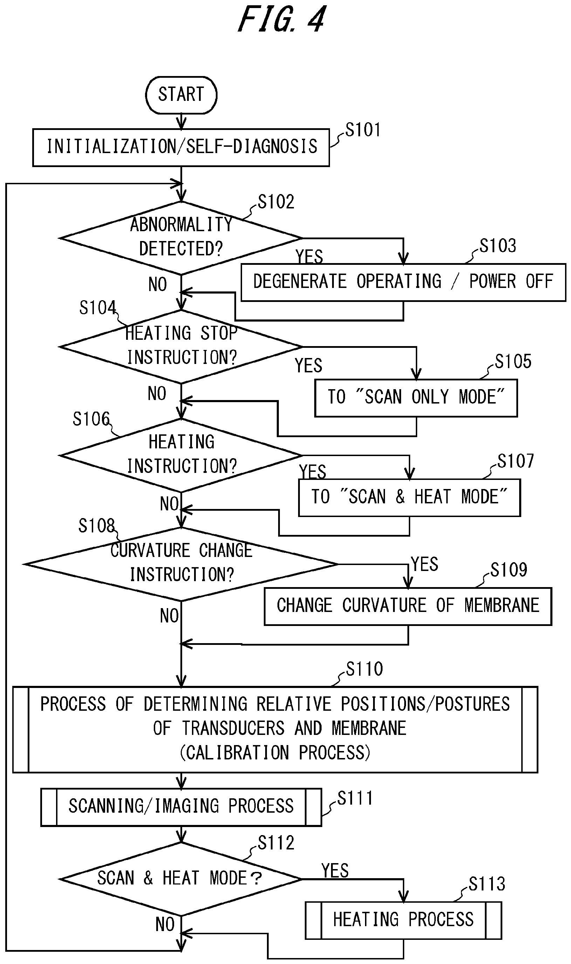

FIG. 4 is a flowchart showing the flow of the startup/operation process in the present embodiment. The process shown in this flowchart starts when the ultrasonic oscillator 1 is powered on.

In step S101, initialization and self-diagnosis are performed. The controller 10 performs self-diagnosis of the device and confirms that the ultrasonic oscillator 1 operates normally. Then, with the activation of the ultrasonic oscillator 1, the controller 10 outputs a control signal prepared in advance to the actuator 14 to control the actuator 14 so that the curvature of the concave portion 111 of the membrane 11 becomes a predetermined initial value.

Specifically, the controller 10 operates the actuator 14 to change the curvature of the concave portion 111 of the membrane 11, and sets the phase of the heating ultrasonic wave oscillated from the ultrasonic transducer 12, to adjust a focal point (heating position) when the ultrasonic waves are oscillated by the ultrasonic transducer array 13 to an initial position.

Thereafter, the ultrasonic oscillator 1 transitions to an operating state in which scanning and heating can be performed, and the process proceeds to step S102. The ultrasonic oscillator 1 in the operating state analyzes the internal structure of the body and provides "inspection / diagnosis aid" to inform the user of the internal structure of the body and heating (cauterization) following the movement of internal organs in the body, while periodically measuring the position and posture of the ultrasonic transducer 12 and the shape of the contact surface.

In steps S102 and S103, an abnormality check of the apparatus is performed, and when an abnormality is detected, the apparatus transits to the degenerate operating state or the power is turned off. In the present embodiment, a state in which scanning and heating can be performed is referred to as an "operating state", and a case where all constituent devices are normally operating is referred to as a "normal operating state" in particular. Depending on the object to be scanned and heated and the operational environment, even if some equipment (for example, some ultrasonic transducers 12 etc.) are malfunctioning, as long as there is no critical problem in scanning/heating, it may be desirable to continue scanning or heating. In this case, the ultrasonic oscillator 1 may perform a degenerate operation in which some functions are stopped and scanning/heating is performed. When the abnormality check of the apparatus is completed, the process proceeds to step S104.

In steps S104 to S107, the operation mode of the ultrasonic oscillator 1 is set according to the operation by the user. While the ultrasonic oscillator 1 is in the operating state (normal operating state or degenerate operating state), the user instructs the controller 10 to heat (oscillation from the ultrasonic transducer 12) the target 9 to be heated by operating the lever 17 when the user checks the image displayed on the display 18 and determines that the state is acceptable for heating. When the user operates the lever 17, a heating instruction is input to the controller 10. In the present embodiment, a heating instruction by the operation of the lever 17 is exemplified, but instructions by the user may be performed via other interfaces. For example, by providing a heating start button and a heating stop button, the user may be able to instruct start and stop of heating.

The controller 10 determines whether there is an input of the heating instruction by the lever operation of the user. When it is determined that the heating stop instruction (see step S609) has been input, the operation mode is set to "scan only mode "(step S105). The heating stop instruction is issued when the user releases the lever 17 or when the misalignment between the heating position and the heating target 9 exceeds a predetermined criterion in a heating process described later. On the other hand, when it is determined that a heating instruction is input (or a heating start instruction is input) ("YES" in step S106), the operation mode is set to "scan & heat mode" (step S107). Thereafter, the process proceeds to step S108.

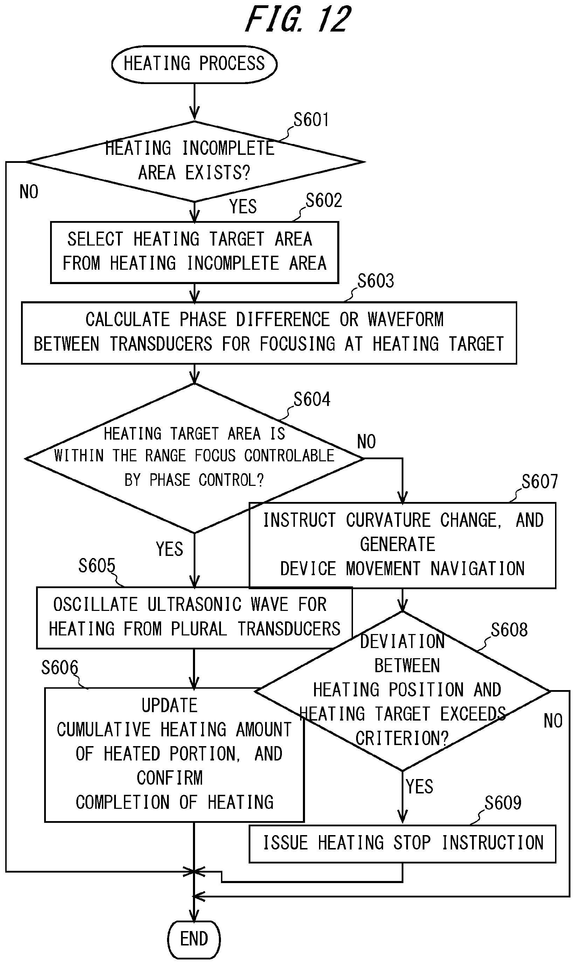

In steps S108 and S109, heating position control by curvature change is performed. When the curvature change instruction is issued by a heating process described later ("YES" of step S108. The process of issuing the curvature change instruction will be described later with reference to FIG. 12), the controller 10 adjusts the heating position (focal position) to arrange a focal point (heating position) of the ultrasonic wave oscillated by the ultrasonic transducer array 13 to be at or near the position of the heating target 9, in accordance with the latest information (image data, diagnostic result, position of the heating target 9) acquired by imaging (step S109).

Here, the adjustment of the heating position is performed by changing the curvature of the concave portion 111 of the membrane 11 under the control of the actuator 14. Specifically, the controller 10 retains in advance a formula or a table in which the correspondence relationship between the curvature of the concave portion 111, the positions and orientations of the ultrasonic transducers 12, and the focal point (heating position) is defined. The controller 10 acquires a required curvature based on the calculated positions and orientations of the respective ultrasonic transducers 12 and a position required to be set as the focal point (the front surface or the inside of the living body 8 including the heating target 9 in the embodiment). Moreover, the controller 10 also retains a formula or a table in which the correspondence relationship between the operation amounts (pushing/pulling amounts) of the respective actuators 14 and the curvature of the concave portion 111 is defined. The controller 10 acquires the operation amounts of the actuators 14 based on the acquired curvature to control the actuators 14. After that, the processing proceeds to step S110.

In step S110, the process of determining the relative positions/postures of the transducers and the membrane shape (hereinafter also referred to as "calibration process") is performed. In the operating state, the "inspection/diagnosis aid" and the "heating process" are needed to be executed after determining the relative positions and postures of the ultrasonic transducers 12 and the shape of the contact portion 112 (contact surface) of the membrane 11 that can change every moment. Therefore, the controller 10 applies a drive voltage to a predetermined number of ultrasonic transducers 12 (oscillation side transducers) among the ultrasonic transducers 12 in the ultrasonic transducer array 13 to cause ultrasonic waves to oscillate. The oscillated ultrasonic wave is sensed (received) as a direct wave and a reflected wave by other ultrasonic transducers 12 not used for oscillation.

The ultrasonic transducer 12 which sensed the ultrasonic wave outputs a sensing signal, and in the case where there is only one ultrasonic transducer 12 that performs oscillation in a certain time slot, upon receiving the sensing signal, the controller 10 can grasp that the sensed ultrasonic wave has been oscillated from the ultrasonic transducer 12 which has been oscillating in the relevant time slot. Even when a plurality of ultrasonic transducers 12 oscillate at the same time, the controller 10 which has received the sensing signal obtains the amplitude, frequency, phase, and the like of the sensed ultrasonic wave from the sensing signal, so that the control unit 10 can grasp which ultrasonic transducer 12 oscillated the sensed ultrasonic wave, based on features such as the amplitude, frequency, phase, time difference between arrival times, and the like of each ultrasonic transducer 12.

Further, regarding the sensed ultrasonic waves oscillated from the same ultrasonic transducer 12, the controller 10 calculates the time (hereinafter referred to as "direct wave propagation time") taken for the direct wave to reach the sensing-side transducer from the oscillation-side transducer, and also calculates the time (hereinafter referred to as "reflected wave propagation time") that the reflected wave took from the oscillation-side transducer to the sensing-side transducer. Based on the information such as the direct wave propagation time, the reflected wave propagation time, the waveform and the intensity of the received wave, which are calculated for each of the plurality of ultrasonic transducers 12, the controller 10 calculates the position and orientation (posture) and the reflection surface (contact surface of the contact portion 112). Thereafter, the process proceeds to step S111.

In step Sill, scanning/imaging process is performed. The controller 10 applies a voltage for oscillation of the imaging ultrasonic wave to the oscillation-side transducer of the ultrasonic transducer array 13 to operate ultrasonic oscillation toward the living body 8, associated detection of the reflected waves, image data generation based on the reflected waves, and storage processing of the image data. Thereafter, the process proceeds to step S112.

In steps S112 and S113, when the operation mode of the ultrasonic oscillator 1 is "scan & heat mode", heating process is performed. When the operation mode of the ultrasonic oscillator 1 is the "scan & heat mode" (YES in step S112), the controller 10 operates the heating of the target 9 to be heated by moving the heating position (focal position) within the area of the heating target 9 and causing the oscillation of the ultrasonic waves for heating by the ultrasonic transducers 12 (step S113). At this time, the controller 10 accumulates information on the cumulative heating amount in the heated area, and the area (heating completion area) in which heating has been completed, among the heating target 9. Thereafter, the process proceeds to step S102.

That is, the ultrasonic oscillator 1 in the operating state periodically (for example, every 0.1 seconds) repeats the processing shown in steps S102 to S113 until the device enters the degenerate operating state or the power of the device is turned off. In this way, the ultrasonic oscillator 1 according to the present embodiment makes it possible to heat the target 9 to be heated while easily adjusting the heating position to an appropriate position.

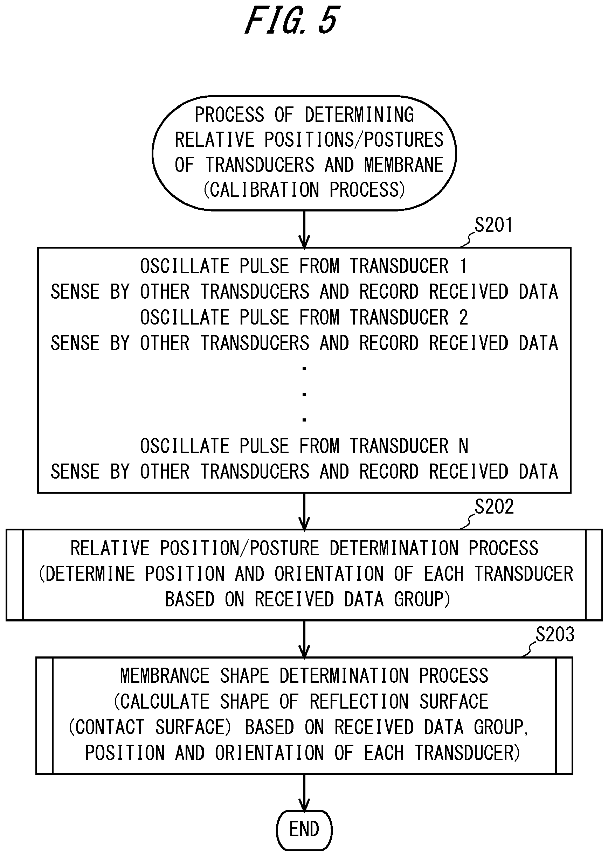

FIG. 5 is a flowchart showing the flow of the process of determining the relative positions/postures of the transducers and the membrane shape (calibration process) in the present embodiment. The process shown in this flowchart is executed in response to the start of step S110 in the startup/operation process shown in FIG. 4. That is, this flowchart explains in detail the process shown in step S110 out of the startup/operation process shown in FIG. 4.

In step S201, pulse oscillation from the ultrasonic transducer 12, sensing by the other ultrasonic transducer 12, and recording of received data are performed. The controller 10 causes the ultrasonic transducers 12 to oscillate ultrasonic waves by sequentially applying a driving voltage one by one to a predetermined number of ultrasonic transducers 12 (oscillation-side transducers) among the ultrasonic transducers 12 in the ultrasonic transducer array 13. The oscillated ultrasonic wave is sensed (received) as a direct wave and a reflected wave by other ultrasonic transducers 12 not used for oscillation. Ultrasonic transducer 12 which senses ultrasonic wave outputs a sensing signal. Here, since there is only one ultrasonic transducer 12 that performs oscillation in a certain time slot, the control unit 10 can recognize that the sensed ultrasonic wave has been oscillated from the ultrasonic transducer 12 that has been oscillating in the certain time slot. Even when a plurality of ultrasonic transducers 12 oscillate at the same time, the controller 10 which has received the sensing signal can recognize which ultrasonic transducer 12 oscillated the sensed ultrasonic wave, based on the characteristics such as amplitude, frequency, phase, time difference between arrival times, and the like for each ultrasonic transducer 12, by obtaining the amplitude, frequency, phase and the like of the sensed ultrasonic wave from the sensing signal.

While changing the ultrasonic transducer 12 used for oscillation, the controller 10 causes a predetermined number of ultrasonic transducers 12 to perform oscillation and causes another ultrasonic transducer 12 to perform sensing. The obtained data is recorded in the RAM or the storage of the controller 10 in association with the oscillated ultrasonic transducer 12, the sensed ultrasonic transducer 12, the oscillation time and the sensing time. Thereafter, the process proceeds to step S202.

In step S202, the position and the orientation (posture) of each ultrasonic transducer 12 are determined. Regarding the sensed ultrasonic waves oscillated from the same ultrasonic transducer 12, the controller 10 calculates the time taken for the direct wave to reach the sensing-side transducer from the oscillation-side transducer based on the received data group (hereinafter referred to as "direct wave propagation time") and calculates the time taken for the reflected wave to reach the sensing side transducer from the oscillation-side transducer (hereinafter referred to as "reflected wave propagation time"). Then, the controller 10 calculates the position and the orientation (posture) of each ultrasonic transducer 12 based on the direct wave propagation time calculated for each of the plurality of ultrasonic transducers 12. Thereafter, the process proceeds to step S203.

In step S203, the shape of the reflection surface (contact surface of the contact portion 112) is determined. Regarding the sensed ultrasonic waves oscillated from the same ultrasonic transducer 12, the controller 10 calculates the time that the one-time reflected wave took from the oscillation-side transducer to the sensing-side transducer (hereinafter referred to as "one-time reflected wave propagation time"). Based on the position and the orientation (posture) of each of the ultrasonic transducers 12 and the reflected wave propagation time calculated in step S202 for each of the plurality of ultrasonic transducers 12, the controller 10 calculates the shape of the reflection surface (contact surface of the contact portion 112). After that, the processing shown in this flowchart ends.

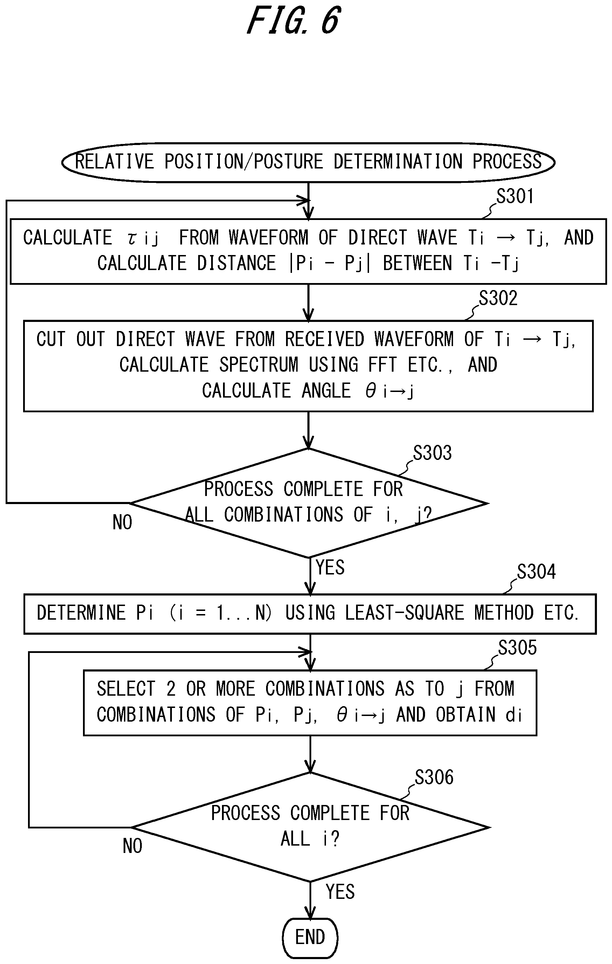

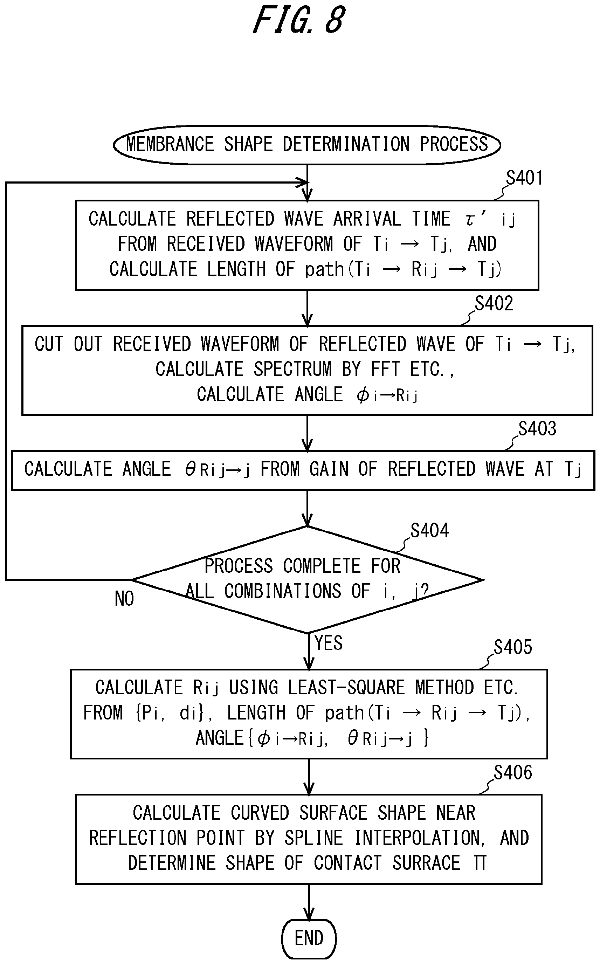

Next, more detailed processing contents of the calibration process explained with reference to FIG. 5 will be described with reference to FIGs. 6 to 9. Here, in the flowcharts of FIGs. 6 and 8, N ultrasonic transducers 12 are shown as $T_i$ (i is a natural number from 1 to N), respectively. The contents of other symbols are as follows.

$P_i$: Coordinate of $T_i$

$d_i$: Orientation (posture) of $T_i$. The direction in which the oscillating surface of the ultrasonic transducer 12 is directed is represented as a vector of size 1.

$\tau_{ij}$: Direct wave propagation time of direct wave among the waves oscillated from $T_i$ and sensed by $T_j$,

$\tau'_{ij}$: Propagation time of the wave reflected one time at the reflection point $R_{ij}$ on the contact surface $\Pi$ among the wave oscillated from $T_i$ and sensed by $T_j$ (one-time reflected wave propagation time)

$b_{ij}$: the normal of $\Pi$ at $R_{ij}$

Note that the coordinate system used here is fixed by setting the rule such as "the specific $T_i$ is the origin, the specific $T_j$ ($j \neq i$) is on the x>0 side of the plane zx".

When calculating (estimating) the relative position/orientation of the transducer and the membrane shape by the processing shown in FIGs. 6 to 9, the following contents are presupposed in advance.

1. The total number N of transducers arranged on the membrane 11 is known.
2. The ultrasonic transducers are arranged in a bowl shape on the concave portion 111 of the membrane 11.
3. The volume of the gel filled in the membrane 11 is known.
4. Membrane 11 expands evenly.
5. The arrangement of the ultrasonic transducers 12 in a state where the membrane 11 is "contracting" is known at the time of manufacture of the membrane 11 with the ultrasonic transducer 12.

Thereby it is possible to determine provisional positions (provisional coordinates) of the N transducers. As for the directions of the transducers, they are assumed that all face a certain point (focal point) (provisional directions). Specifically, the provisional directions are set as follows by assuming that all the transducers are oriented to the center C of the spherical surface when the curved surface is approximated to a spherical surface.

$$d_i = \frac{C - P_i}{|C - P_i|}$$

FIG. 6 is a flowchart showing the flow of the relative position/posture determination process of the transducers according to the present embodiment. The process shown in this flowchart is executed in response to the start of step S202 in the calibration process shown in FIG. 5. That is, this flowchart explains in more detail the processing shown in step S202 out of the calibration processing shown in FIG. 5.

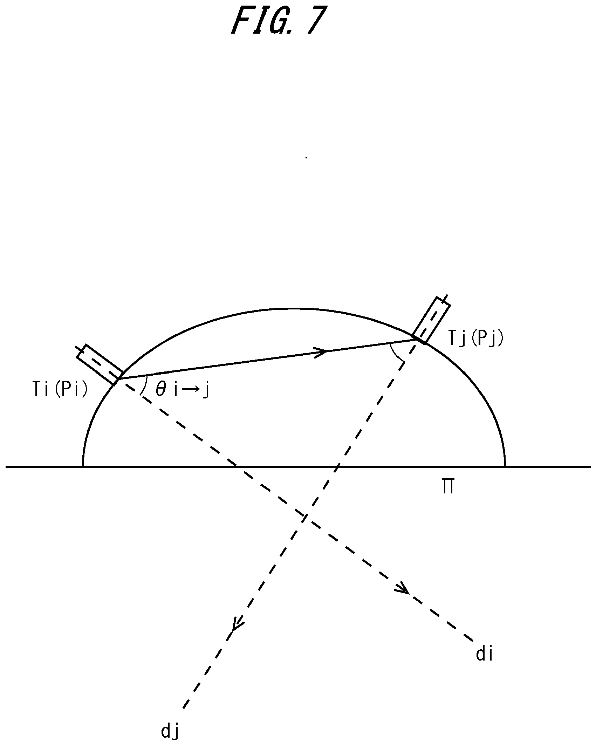

FIG. 7 is a diagram showing the relationship between the orientation (posture) of the oscillation-side transducer and the sensing-side transducer and the direct wave oscillated from the oscillation-side transducer and sensed by the sensing-side transducer in the present embodiment. In the example shown in FIG. 7, among the ultrasonic waves oscillated from the oscillation-side transducer $T_i$ (coordinates $P_i$), the ultrasonic waves propagating in the direction forming the angle $\theta_{ij}$) with the direction $d_i$ of $T_i$ are sensed by the sensing-side transducer $T_j$ (coordinates $P_j$).

In step S301, the distance between $T_i$ and $T_j$ is calculated. The controller 10 identifies $\tau_{ij}$ from the waveform of the direct wave out of the received data of the pulse oscillated from $T_i$ and sensed by $T_j$ acquired in step S201. It is possible to specify the direct wave by searching for pulses observed at $T_j$ near the predicted propagation time of the ultrasonic wave oscillated from $T_i$ directly reached $T_j$ ($T_{ij}$, expected = $l_{ij}$ / c), which is possible to be calculated based on the provisional path length ($l_{ij} = |P_j - P_i|$) of the direct wave calculated from the provisional coordinates $P_i$, $P_j$, and the sound velocity c (in the present embodiment, the sound wave propagation speed in the gel) in the membrane 11.

Then, the controller 10 calculates the distance $|P_i - P_j|$ between $T_i$ and $T_j$ from the calculated $\tau_{ij}$ and the calculated sound velocity c (the distance is $|P_i - P_j| = c * \tau_{ij}$, as the product of the sound velocity c and the propagation time $\tau_{ij}$). Thereafter, the process proceeds to step S302.

In step S302, the visual angle $\theta_{ij}$ between the center axis of oscillation and $T_j$ viewed from $T_i$ is calculated. As described above, the "visual angle" is an angle indicating how many degrees the point (the object such as another transducer or reflection point, etc.) deviates from the center axis of oscillation extending in the orientation $d_i$ of the transducer. For example, the visual angle $\theta_{ij}$ between the center axis of oscillation and $T_j$ viewed from $T_i$ is an angle formed by "the direction of the center axis passing through the oscillation plane of $T_i$ (the center direction in which the ultrasonic wave oscillated from $T_i$ is directed)" and "a line connecting the center of the oscillation plane of the $T_i$ and the center of the sensing surface of $T_j$" (see FIG. 7).

The controller 10 cuts out the waveform of the direct wave from the waveform of the received wave out of the received data of the pulse oscillated from $T_i$ and sensed by $T_j$, finds spectra using frequency-domain representation (for example, Fast Fourier Transform, Wavelet transform, etc.) and calculates the visual angle $\theta_{ij}$ of the center axis of oscillation and $T_j$ viewed from $T_i$. The ultrasonic pulse signal sent from $T_i$ to $T_j$ includes harmonics of integral multiples other than the fundamental frequency. In the Fraunhofer region, the directivity differs depending on the wavelength, so the harmonic component ratio of the direct wave pulse from $T_i$ observed at $T_j$ depends on the direction $d_i$ of $T_i$ and the visual angle $\theta_{ij}$. Therefore, the controller 10 can estimate $\theta_{ij}$ by comparing the $\theta$ dependency of the component ratio calculated or measured in advance with the harmonic component ratio observed by $T_j$. As the wave propagates through the medium, the attenuation of the amplitude occurs due to the viscosity and the like of the medium, and the attenuation rate also depends on the frequency of the wave. If the medium is decided, the frequency dependency of the attenuation factor is known, and if the propagation distance is known, the attenuation during propagation in the medium is calculated for each frequency and the attenuation by the medium can be corrected. Thereafter, the process proceeds to step S303.

In step S303, it is determined whether or not the processing in step S301 and step S302 has been completed for the combinations capable of calculating an angle out of combinations of i and j (i and j are natural numbers from 1 to N) with which the controller 10 can determine the arrival time or frequency expression of the direct wave from $T_i$ to $T_j$. When there is a combination of the ultrasonic transducers 12 for which the calculation of the distance between the ultrasonic transducers 12 and the calculation of the visual angle has not been completed, the controller 10 updates i and j to incomplete combination, and returns to step S301. On the other hand, if it is determined that the calculation of the distance between the ultrasonic transducers 12 and the calculation of the visual angle with respect to the combinations capable of calculating the angle are completed, the process proceeds to step S304.

In step S304, the position (coordinates $P_i$ of $T_i$) of each ultrasonic transducer 12 is calculated. The controller 10 calculates the coordinates $P_i$ (i is a natural number from 1 to N) of $T_i$, by using a fitting algorithm for data including errors, the fitting algorithm using the distance between the ultrasonic transducers 12 calculated based on the provisional coordinates and the provisional direction, the visual angle between the center axis of oscillation and the other ultrasonic transducer 12 viewed from the ultrasonic transducer 12, the distance between the ultrasonic transducers 12 calculated in the processing from step S301 to step S303 based on the data obtained by actual pulse oscillation/ sensing ($|P_i - P_j|$), and the visual angle between the center axis of oscillation and the other ultrasonic transducer 12 viewed from the ultrasonic transducer 12 ($\theta_{ij}$), as parameters. The fitting algorithm used here is not limited. For example, it is possible to use a least-square method (Lovenberg-Marquardt method, etc.), kernel regression, or the like. According to these algorithms, it is also possible to estimate the error of the calculated coordinates $P_i$ (estimated value). Thereafter, the process proceeds to step S305.

In step S305, the orientation (posture) $d_i$ of the ultrasonic transducer $T_i$ is calculated. The controller 10 selects two or more combinations as to j from the combinations of $P_i$, $P_j$, $\theta_{ij}$, and obtains $d_i$. In the processing so far, $\theta_k$, $\theta_l$, ... are also calculated for the ultrasonic transducers Tk, Tl, ... which are distant from the $T_i$ (Fraunhofer region) and different from $T_j$. Therefore, angles $\theta_j$, $\theta_k$, $\theta_l$, ... formed by the orientation $d_i$ of $T_i$ and i $\rightarrow$ j, i $\rightarrow$ k, i $\rightarrow$ 1, ... can be found. At this time, since cos $\theta_j$ is obtained by the following expression, if two or more independent conditions (for example, a set of i, j and a set of i, k) are given, the direction $d_i$ of the transducer can be determined (latitude is 2). Thereafter, the process proceeds to step S306.

$$d_i \cdot \frac{P_j - P_i}{|P_j - P_i|} = cos\, \theta_j$$

In step S306, it is determined whether or not the process of step S305 has been completed for each of i (1 to N natural numbers) capable of calculating the orientation $d_i$. When there is an ultrasonic transducer 12 whose calculation of the orientation $d_i$ of the transducer has not been completed, the controller 10 updates i to the number of the incomplete ultrasonic transducer 12, and returns the process to step S305. On the other hand, if it is determined that calculation of the direction $d_i$ for the calculable ones of the ultrasonic transducers 12 is completed, the process shown in this flowchart ends.

According to the process of determining the relative position/posture of the transducer described above, the position and orientation {$P_i$, $d_i$} (i is a natural number from 1 to N) are calculated for $T_i$ which is calculable the position and orientation. After that, the membrane shape determination process is subsequently executed.

FIG. 8 is a flowchart showing the flow of the membrane shape determination process in the present embodiment. The process shown in this flowchart is executed with the start of step S203 in the calibration process shown in FIG. 5. That is, this flowchart explains in more detail the processing shown in step S203 out of the calibration process shown in FIG. 5. For this reason, the positions and orientations {$P_i$, $d_i$} (i is a natural number from 1 to N) have already been calculated for calculable $T_i$ before executing the process shown in this flowchart.

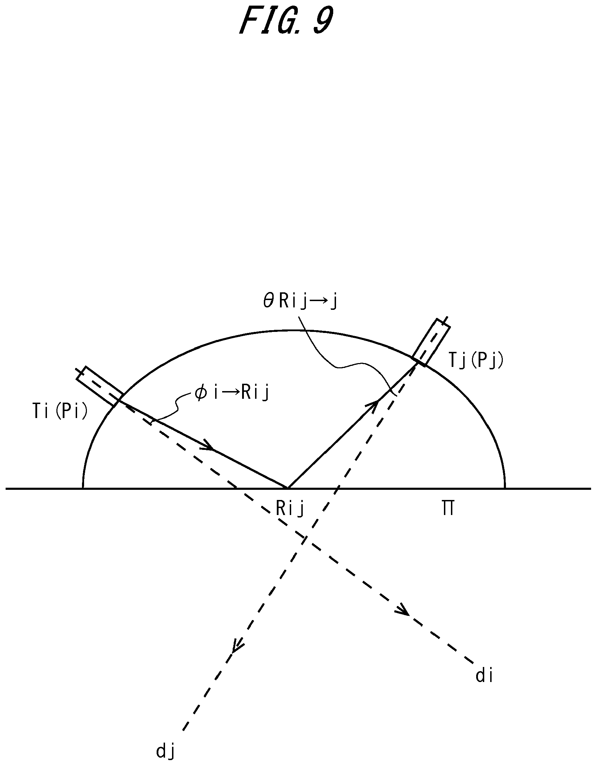

FIG. 9 is a diagram showing the relationship between the orientation (posture) of the oscillation-side transducer and the sensing-side transducer and the reflected wave oscillated from the oscillation-side transducer and sensed by the sensing-side transducer in the present embodiment. In the example shown in FIG. 9, the ultrasonic wave oscillated from the oscillation-side transducer $T_i$ (coordinates $P_i$) propagates in the direction forming the angle $\varphi_{i \rightarrow Rij}$ with the orientation $d_i$ of $T_i$, reflects at the reflection point $R_{ij}$, incidents on the receiving transducer $T_j$ from a direction forming the angle $\theta_{Rij \rightarrow j}$ with the direction $d_j$ of the receiving transducer $T_j$ (coordinates $P_j$), and is received by the receiving transducer $T_j$.

In step S401, the length of the path of the one-time-reflected wave from $T_i$ to $T_j$ is calculated. The controller 10 identifies $\tau'_{ij}$ out of the waveform of the one-time-reflected wave among the received data of the pulse oscillated from $T_i$ and sensed by $T_j$ and acquired in step S201. Here, the length of the path of the one-time-reflected wave is calculated for the case where the ultrasonic pulse signal oscillated from the transducer $T_i$ is reflected at the point $R_{ij}$ on the contact surface $\Pi$ and is observed by the transducer $T_j$ (see FIG. 9). Pulses oscillated from $T_i$ are affected by the directivity on the oscillation side, the reflectivity, and the directivity of the sensing side in addition to the attenuation depending on the path length of $T_i \rightarrow R_{ij} \rightarrow T_j$ when observed at $T_j$, as it propagates through the medium and reaches the sensing-side transducer, the pulses oscillated from $T_i$ attenuate and deform the waveform. Here, the directivity on the oscillation side affects the spectrum, and the directivity on the sensing side influences the intensity (sensitivity) of the sensed pulse (there is no influence on the spectrum or can be ignored). Further, the reflectance is determined from the acoustic impedance of the medium and the contact portion 112 (contact surface).

Then, when the ultrasonic pulse signal oscillated from $T_i$ (oscillated at time t0) is observed at $T_j$, the following pulse signals should be observed at $T_j$, in order of arrival time.

(1) Direct wave of $T_i \rightarrow T_j$
(2) One-time reflected wave of $T_i \rightarrow R_{ij} \rightarrow T_j$
(3) Multiple reflected waves in the membrane 11, reflected waves reflected inside the body

Therefore, in general, among the waveforms sensed by $T_j$, it is estimated that the strongest reflected wave excluding direct waves is a once reflected wave. However, in reality, depending on the combination of the curvature of the concave portion 111 and i, j, the direct wave or the one-time-reflected wave may be too weak to be visible or may not be separated due to overlapping. For such a case, it may be referred to what was investigated about the propagation time, intensity and separation possibility of direct wave or one-time-reflected wave when experiment and trial run in advance. With regard to direct waves, presence or absence of a direct wave can be determined by checking the presence or absence of a waveform sensed at a time close to the expected propagation time of a direct wave between $T_i$ and $T_j$

(see step S301).

Then, the controller 10 obtains $\tau'_{ij}$ based on the identified peak of the one-time-reflected wave, and calculates the path length of the one-time-reflected wave from $T_i$ to $T_j$ ($T_i \rightarrow R_{ij} \rightarrow T_j$) based on $\tau'_{ij}$ and the sound velocity c. The calculation method is the same as the processing in step S301 in which the length of the direct wave path is calculated. Thereafter, the process proceeds to step S402.

In step S402, the visual angle $\varphi_{i \rightarrow Rij}$ between the center axis of oscillation and the reflection point $R_{ij}$ viewed from $T_i$ is calculated. The controller 10 cuts out the waveform of the one-time-reflected wave from the waveform of the received wave among the received data of the pulse oscillated from $T_i$ and sensed by $T_j$, obtains spectra using frequency-domain representation (for example, Fast Fourier Transform, Wavelet transform, etc.) and calculates the visual angle $\varphi_{i \rightarrow Rij}$ between the center axis of oscillation and the reflection point $R_{ij}$ viewed from $T_i$. The calculation method is the same as the processing in step S302. Thereafter, the process proceeds to step S403.

In step S403, the incident angle $\theta_{Rij \rightarrow j}$ to the $T_j$ of the one-time-reflected wave is calculated. The controller 10 calculates the incident angle $\theta_{Rij \rightarrow j}$ to $T_j$ based on the intensity of the sensing pulse and the reflectance on the contact surface, among the received data of the pulse oscillated from $T_i$ and sensed by $T_j$. When pulses of the same intensity arrive at the sensing-side transducer, the intensity of the signal sensed when the incident angle is vertical to the sensing face of the sensing-side transducer is high and the intensity becomes lower as the incidence angle is away from the vertical. Therefore, the controller 10 compares the estimated intensity in the case where the sensing-side transducer senses the pulse vertically with the intensity of the actually received sensing signal, and calculates the incident angle based on the difference. The estimated intensity can be calculated by correcting the estimated intensity calculated based on the emission angle from the oscillation-side transducer, the frequency, the attenuation rate of the medium, the propagation distance, and the like using the reflectance at the contact surface. Thereafter, the process proceeds to step S404.

In step S404, it is determined whether or not the processing of steps 401 to step S403 has been completed for the combinations of i and j which are capable of calculating a combination of $\varphi_{i \rightarrow Rij}$ and $\theta_{Rij \rightarrow j}$ (i and j are natural numbers from 1 to N). When there is a combination of the ultrasonic transducers 12 whose calculation has not been completed, the controller 10 updates i and j to incomplete combination and returns the process to step S401. When it is determined that the calculation is completed for all calculable combinations, the process proceeds to step S405.

In step S405, the coordinates of the reflection point $R_{ij}$ are calculated. The controller 10 calculates $R_{ij}$ based on the data of $\{P_i, d_i\}$, $\{P_j, d_j\}$, the length of the path ($T_i \rightarrow R_{ij} \rightarrow T_j$), and the visual angle $\{\varphi_{i \rightarrow Rij}, \theta_{Rij \rightarrow j}\}$ of the path, obtained in the processing up to step S404. Specifically, it can be calculated using the following equation.

$$\left|\overrightarrow{P_i R_{ij}}\right| + \left|\overrightarrow{R_{ij} P_j}\right| = l_{Ti \rightarrow Rij \rightarrow Tj}$$

$$\frac{\overrightarrow{P_i R_{ij}}}{\left|\overrightarrow{P_i R_{ij}}\right|} \cdot d_i = cos\,\phi_{i \rightarrow Rij}$$

$$\frac{\overrightarrow{P_j R_{ij}}}{|\overrightarrow{P_j R_{ij}}|} \cdot d_j = cos\,\theta_{\,Rij \rightarrow j}$$

Since all the observed values and the values estimated from the observed values used to calculate $R_{ij}$ include errors, the position of $R_{ij}$ may be determined using a fitting algorithm such as a least-square method, etc. to evaluate the error. Also, since three positions of $T_i$, $R_{ij}$, $T_j$ are obtained, it is possible to obtain a vector bisecting the angle $T_i$ $R_{ij}$ $T_j$ on the plane on which three points $T_i$, $R_{ij}$, $T_j$ exist. Since this vector corresponds to the normal $b_{ij}$ of the "tangent plane" characterizing the curved surface in the vicinity of the reflection point $R_{ij}$, also the tangential plane at the reflection point is obtained. By repeating the above process with changing i and j, the coordinates of up to (1/2)N(N-1) reflection points and the tangent plane at the reflection point are obtained. Thereafter, the process proceeds to step S406.

In step S406, the shape of the contact surface $\Pi$ is calculated. As described above, the coordinates of up to (1/2)N(N-1) reflection points and the tangent plane are obtained by the processing so far. Therefore, based on this information, the controller 10 obtains the shape of the contact surface $\Pi$ by using polynomial interpolation, spline interpolation or the like to obtain the curved surface shape near the reflection point. Specifically, the controller 10 obtains the shape of the contact surface $\Pi$ using the following procedure, for example.

(1) Select one reflection point R

(2) Select multiple reflection points near R in ascending order of distance from R

(3) A function that reproduces a curved surface in the vicinity of R is determined when assuming that the surface in the vicinity of R can be approximated by a 2-3 order polynomial of (x, y, z), a spline function etc., and setting the condition that the curved surface passes through R and its vicinity point and passes through the tangent plane near that point.

The interpolation function may be determined using many points without using the normal line information, or the error of the interpolation function may be estimated from the error information of the reflection point. Thereafter, the process shown in this flowchart ends.

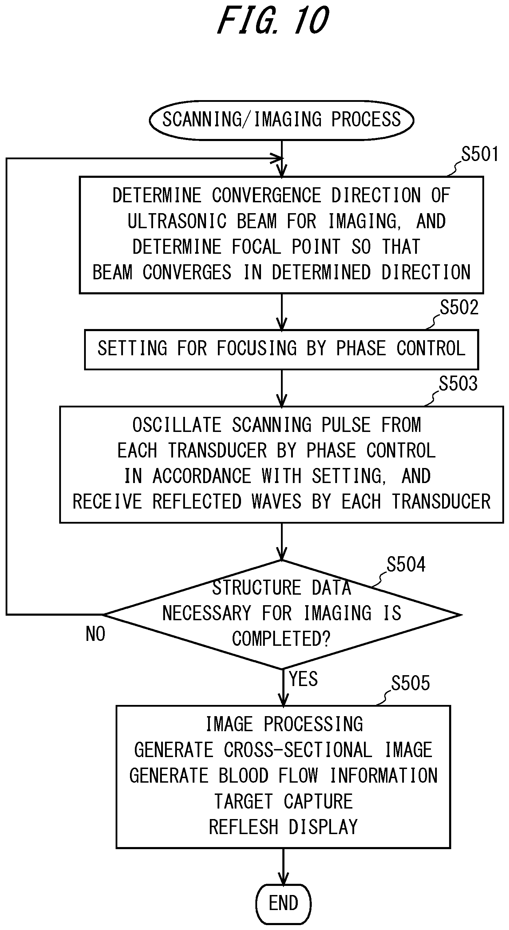

FIG. 10 is a flowchart showing the flow of scanning and imaging process in the present embodiment. The process shown in this flowchart is executed in response to the start of step S111 in the startup/operation process shown in FIG. 4. That is, this flowchart explains in more detail the processing shown in step Sill out of the startup/operation process shown in FIG. 4.

In step S501, the focus of the ultrasonic beam oscillating for imaging is determined. The controller 10 determines the convergence direction of the ultrasonic beam for imaging based on the area to be imaged and determines the position (coordinates) of the focal point so that the beam converges in the determined direction. Thereafter, the process proceeds to step S502.

In step S502, calculation for focusing by phase control is performed. In order to control the phases of the waves propagated from each transducer at a specific point so as to strengthen each other, the propagation time from each transducer to the target is estimated so that the wave propagating from each transducer becomes a state (of specific phase) in which the amplitude increases when passing through the target (focal point).

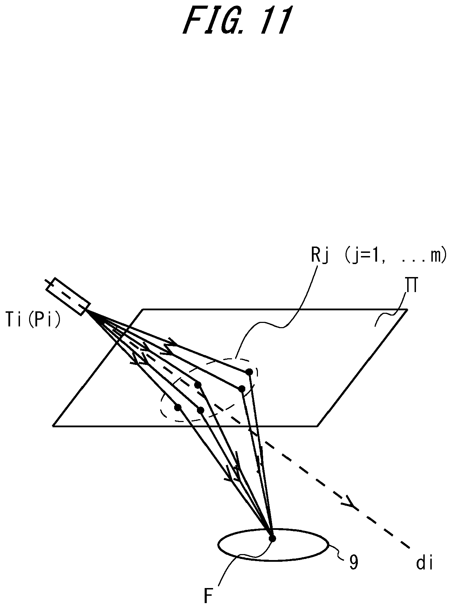

FIG. 11 is a schematic diagram showing calculation contents for focusing by phase control in the present embodiment. The set of points (reflection points and points interpolated between reflection points) already obtained on the contact surface is defined as {Rj: j = 1,... M}. Also, the route of $T_i \rightarrow$ Rj is propagation having normal directivity, and Rj $\rightarrow$ F is a spherical wave whose source is Rj. Then, the controller 10 obtains the waveform (amplitude, phase, propagation time) of a wave oscillating from $T_i$, passing through the contact surface and passing through the target F (see FIG. 11), by adding up the wave propagated through the path of $T_i \rightarrow$ Rj $\rightarrow$ F(i is a natural number from 1 to n. j is a natural number from 1 to m), which is obtained from the path length 1 $T_i \rightarrow$ Rj $\rightarrow$ F of the path $T_i \rightarrow$ Rj $\rightarrow$ F and the directivity factor P ($\theta_{ij}$), for all routes Rj (j is a natural number from 1 to m). In this manner, the controller 10 obtains the waveform (amplitude, phase, propagation time) of the wave passing through the target F with respect to the ultrasonic transducer 12 used for oscillation.

Thereafter, the controller 10 adjusts the oscillation time (phase) between the plurality of oscillation-side transducers so that the amplitudes of the waves oscillated from the plurality of oscillation-side transducers are the largest in the target F. Specifically, the control unit 10 defines $\tau_i$ as the time from the oscillation to the timing when the amplitude of the wave passing through F becomes the maximum when $\tau_i$ emits a sharp pulse from $T_i$, sets the oscillation time (phase) such that the oscillation time (phase) of the other transducers delay with respect to the oscillation side transducer at which $\tau_i$ becomes the maximum, and the timing at which the amplitude becomes maximum coincides at F. By doing in this way, the passing time at which the amplitude becomes the largest matches for the waves from the plurality of transducers passing through the target F, and the target becomes the focal point.

In the present embodiment, the case where the medium is changed on one surface (the medium contains two layers of the gel and the living body 8) has been described (see FIG. 11). However, in the case where the medium is changed to two or more surfaces, if the shape of the surface on which the medium is changed can be obtained, focusing by phase control can be performed by the same processing. In this case, it is also possible to estimate the shape of the second and subsequent surfaces by using the above-described contact surface shape determination method. That is, according to the present disclosure, it is possible to perform focus control even for an object that has a multilayer structure and repeats refraction complexly. Thereafter, the process proceeds to step S503.

In step S503, oscillation of the scanning pulse is performed. The controller 10 performs phase control as set in step S502 and causes each ultrasonic transducer 12 to oscillate the scanning pulse. Further, the reflected wave of the oscillated pulse is sensed by each ultrasonic transducer 12. Upon receiving the ultrasonic wave, the ultrasonic transducer 12 outputs a sensing signal, and the controller 10 receives the sensing signal and obtains the amplitude, the frequency, the phase and the like of the sensed ultrasonic wave from the sensing signal. The obtained data is recorded in the RAM or the storage of the controller 10. Thereafter, the process proceeds to step S504.

In step S504, it is determined whether or not the structure data necessary for imaging is completed. If there is an unscanned area among the areas to be imaged, the process returns to step S501. On the other hand, if all the scans of the range to be imaged are completed and necessary data is obtained, the process proceeds to step 505.

In step S505, image processing is performed. The controller 10 amplifies and digitizes sensing signals output from ultrasonic transducers 12 having sensed reflected waves to generate three-dimensional image data and cross-sectional image inside the living body 8 including the heating target 9 based on the reflected waves. For the generation of three-dimensional image data using reflected waves, a related art used in an echographic investigation or the like may be employed. In addition, the controller 10 specifies a position in the three-dimensional image data corresponding to the coordinates of the heating position (focal position) based on the curvature of the current membrane 11, the setting of the phases of the ultrasonic waves for heating, or the like. Furthermore, the controller 10 updates the display content of the display 18 based on the generated cross-sectional image and the specified target data.

At this time, target capture (identification of the target to be heated 9) and display based on blood flow information or image diagnosis may be performed. Conventional techniques used for echographic investigation and the like can also be adopted for target capture by blood flow information or image diagnosis. The controller 10 specifies the heating target 9 in the image and stores information on the position of the heating target 9 in the image together with the image data.

In addition, the controller 10 generates two-dimensional image data based on the three-dimensional image data and further specifies the position of the heating target 9 and the heating position (focal position) in the two-dimensional image data. The heating position in the two-dimensional image data may be specified in such a way that the controller 10 projects the heating position in the three-dimensional image data onto the two-dimensional image data. Then, the controller 10 outputs the generated two-dimensional image data together with a display showing the position of the heating target 9 and the focal position in the two-dimensional image data and causes the same to be displayed on the display 18. Thereafter, the process shown in this flowchart ends.

Here, the specification of the heating target 9 in two-dimensional image data may be performed based on an input by a user when the processing of step S505 is performed for the first time after the processing shown in the flowchart starts. When the processing of step S505 is performed for the second and subsequent times, the specification of the heating target 9 in two-dimensional image data may be performed based on the comparison (matching) between image data acquired when the processing of step S505 is previously performed and image data acquired when the processing of step S505 is performed this time.

More specifically, when the processing of step S505 is performed for the first time, the controller 10 causes two-dimensional image data to be displayed on the display 18 and receives a designating operation by the user who has confirmed a displayed image to specify the position of the heating target 9 in the image data. Here, the designating operation by the user represents, for example, a touching operation in which the user touches the displayed position of the heating target 9 on the display when the display 18 is a touch-screen type display. The controller 10 stores the position of the heating target 9 in the image data designated by the user and the characteristics of the image. The embodiment employs as an example a mode in which the heating target 9 is specified based on the designation by the user. However, other techniques may be employed to specify the heating target 9. For example, it may be possible to employ a mode in which the heating target 9 is automatically specified by the controller 10 based on the brightness, tone, or the like of the respective pixels of image data.

When the processing of step S505 is performed for the second and subsequent times, the controller 10 compares (matches) image data newly generated by imaging with previous image data. Thus, the controller 10 determines which position of the newly-generated image data corresponds to the heating target 9 specified in the previous image data and specifies the position, which has been determined to be the position corresponding to the heating target 9 specified in the previous image data, as the heating target 9 in the newly-generated image data.

FIG. 12 is a flowchart showing the flow of the heating process in the present embodiment. The process shown in this flowchart is executed upon the start of step S113 in the startup/operation process shown in FIG. 4 as a trigger. That is, this flowchart explains in more detail the processing shown in step S113 out of the startup/operation process shown in FIG. 4.

In steps S601 and S602, the heating target area is selected when there is an area where heating is incomplete. The controller 10 compares the entire area of the heating target 9 in the held image data with the heating completion area accumulated in the previous processes to determine whether or not there is a region where heating has not been completed in the heating target 9 (Step S601).

If it is determined that there is no remaining unheated area (the entire heating target 9 is completely heated), the process shown in this flowchart ends. On the other hand, if it is determined that an unheated area remains, the controller 10 selects a next heating target area to be heated from the unheated area (step S602). Here, for example, the controller 10 selects a heating target area so that the heating position (focal position) moves within the area of the heating target 9. Thereafter, the process proceeds to step S603.

In step S603, a phase difference or waveform between the ultrasonic transducers 12 for focusing on the heating area is calculated, and focus control using phase control or phase conjugation method or the like is performed. Details of the processing are the same as the step S502 described with reference to FIG. 10. Thereafter, the process proceeds to step S604.

In step S604, it is determined whether or not the heating target area is within the focus controllable range by the phase control. The controller 10 determines whether or not the heating position (focal position) can be adjusted to the position of the heating target 9 by the focus control calculated in step S603. When the heating target area is not within the focus controllable range, in other words, when the deviation between the heating position and the heating target 9 is larger than the upper limit of the range that can be resolved by focus control of the ultrasonic wave oscillated from the ultrasonic transducer 12, the process proceeds to step S607. On the other hand, if the heating target area is within the focus controllable range, the process proceeds to step S605.

In step S605, oscillation of the heating ultrasonic wave is performed. Upon receipt of the input of the heating instruction, the controller 10 performs focus control as set in the processing from step S602 to step S604, and causes the ultrasonic transducers 12 to oscillate ultrasonic waves for heating so that the heating target 9 is heated.

In step S605, the controller 10 may control each of the plurality of ultrasonic transducers 12 to adjust the phases of the ultrasonic waves oscillated from each of the ultrasonic transducers 12, whereby part of the oscillated ultrasonic waves is canceled and the area heated by ultrasonic waves is limited. More specifically, when the size of the heating target 9 is smaller than the heating area centering on the focal point, the control unit 10 causes some of the ultrasonic transducers 12 to oscillate ultrasonic waves that are in opposite phases to the ultrasonic waves oscillated from the other ultrasonic transducers 12 in a region where heating is not desired. As the phase is controlled by the controller 10 as described above, ultrasonic waves cancel each other in a region where heating is not desired, and heating is canceled.

That is, according to the ultrasonic oscillator 1 of the present embodiment, even when the size of the heating target 9 is smaller than the heating area centered on the focal point, it is possible not to heat the area other than the heating target 9 while heating the heating target 9, by canceling the heating in a part of the area. Thereafter, the process proceeds to step S606.

In step S606, the cumulative heating amount of the heated portion is updated, and it is confirmed whether or not the heating of the relevant portion is completed. The controller 10 updates the cumulative heating amount for the area in which heating has been performed among the heating target 9. Then, when the cumulative heating amount of the part becomes equal to or higher than the predetermined heating amount, the part is set to the "heating completion area". Thereafter, the process returns to step S601.

In step S607, a curvature change instruction for focusing and a device movement navigation generation are performed. When it is determined that the focus control is not possible within the range in which focus control by phase control (NO in step S604), the controller 10 issues an instruction to change the curvature of the concave portion 111 of the membrane 11. Note that the control for actual curvature changing is performed in the above-described step S109 in response to the curvature change instruction.

In addition, the controller 10 compares the position of the heating target 9 with the heating position (focal position) in the image data and generates and outputs instructing information for guiding the heating position to the position of the heating target 9. More specifically, the controller 10 compares the heating target 9 with the heating position in the image data in a coordinate system to calculate the deviation between the focal position and the heating target 9. Then, when the deviation between the focal position and the heating target 9 exists, the controller 10 plots and outputs instructing information such as an arrow that connects the focal position in the image data and a predetermined position (for example, a position of center of gravity in the area of the heating target 9) included in the area of the heating target 9, and then causes the display 18 to display the instructing information so as to be overlapped with an image based on the image data. The user sees the instructing information displayed on the display 18, determines a direction and a movement amount for moving the ultrasonic oscillator 1 based on, for example, the direction, length, or the like of the arrow, and operates the handle 16 to move the ultrasonic oscillator 1 such that the concave surface of the membrane 11 is set at an appropriate position and an appropriate angle. After that, the processing proceeds to step S608.

In steps S608 and S609, a heating stop instruction is issued when the deviation between the heating position and the heating target 9 exceeds a predetermined criterion. The controller 10 compares the position of the heating target 9 with the heating position (focal position) in the image data to determine whether the deviation between the position of the heating target 9 and the heating position exceeds the predetermined criterion (step S608). Here, the predetermined criterion represents the upper limit of a range within which the deviation between the heating position and the heating target 9 is correctable by the change of the curvature of the concave portion 111 of the membrane 11 according to the control of the actuators 14 and the control of the phases of the ultrasonic waves oscillated from the ultrasonic transducers 12.

When it is determined that the deviation exceeds the predetermined criterion, the controller 10 issues a heating stop instruction (step S609). When a heating stop instruction is issued, the operation mode of the ultrasonic oscillator 1 is changed from "scan & heat mode" to "scan only mode" (see step S105 in FIG. 4). That is, when it is determined that the deviation between the position of the heating target 9 and the heating position is larger than the predetermined criterion, the controller 10 stops the oscillation of the ultrasonic waves for heating by the plurality of ultrasonic transducers 12. Further, at this time, the controller 10 may display a warning on the display 18. In the present embodiment, the oscillation is stopped by stopping the power supply to the ultrasonic transducer 12. On the other hand, if it is determined that the deviation is within the predetermined criterion, the process shown in the flowchart ends.

According to the process shown in the present embodiment, the heating automatically resumes when the user operates the handle 16, while operating the lever, to adjust the heating position so as to fall within the area of the heating target 9 (or fall within an automatically adjustable area). However, when the heating position is not corrected into an appropriate position even if predetermined time (for example, a few seconds) elapses, the processing shown in the flowchart may automatically end regardless of the lever operation by the user.

Unlike conventional devices, the ultrasonic oscillator 1 according to the present embodiment does not require means for moving and controlling the ultrasonic transducers 12 with high accuracy. With the ultrasonic oscillator 1 according to the present embodiment, it is possible to accurately and easily heat the heating target 9 by assisting the adjustment made by the user with the curvature change and phase control of the membrane 11.

## Claims

1. An ultrasonic oscillator that heats a target using ultrasonic waves, the ultrasonic oscillator comprising:

   a plurality of transducers that oscillates and/or senses the ultrasonic waves;
   a retention portion that retains the plurality of transducers on a deformable concave portion forming a concave thereof opposing the heating target, so as to retain the plurality of transducers movable in conformity with the concave portion deforming in a state where the plurality of transducers is arranged along the concave; and
   a controller that controls the transducers so as to oscillate the ultrasonic waves,
   wherein the controller comprises:

   receiver that receives sensing signals output from the transducers which sensed the ultrasonic waves oscillated from any of the plurality of transducers,
   a direct wave specifying unit that specifies a sensing signal related to a direct wave included in the received sensing signals,
   a distance calculation unit that calculates the distance between the oscillation side transducer and the sensing side transducer based on time from oscillation to sensing of the direct wave,
   an angle calculation unit that calculates a visual angle between a center axis of oscillation and the sensing side transducer viewed from the oscillation side transducer, based on the sensing signal related to the direct wave, and
   a correction unit that corrects position information indicating a relative positional relationship between the plurality of transducers by using the distance and the visual angle.

2. The ultrasonic oscillator according to claim 1, wherein the controller further comprises a posture calculation unit that calculates an orientation of the oscillation side transducer based on a visual angle between the center axis of oscillation and a first sensing side transducer viewed from the oscillation side transducer, a visual angle between the center axis of oscillation and a second sensing side transducer viewed from the oscillation side transducer, position information of the oscillation side transducer, position information of the first sensing side transducer, and position information of the second sensing side transducer.

3. The ultrasonic oscillator according to claim 2, further comprising
a reflected wave specifying unit that specifies a sensing signal relating to a reflected wave included in the received sensing signals,
wherein the distance calculation unit further calculates the path length of the reflected wave from the oscillation side transducer to the sensing side transducer based on time from oscillation to sensing of the reflected wave, and
the angle calculation unit further calculates a visual angle between the center axis of oscillation and a reflection point viewed from the oscillation side transducer, based on the sensing signal related to the reflected wave, and further calculates the incident angle from the reflection point to the sensing side transducer based on the intensity of the reflected wave.

4. The ultrasonic oscillator according to claim 3, wherein
the target is included in a living body,
the retention portion is a hollow member having the concave portion and a contact portion that contacts the living body and seals an opening of the concave portion, and
the controller further comprises a reflection point calculation unit that calculates reflection point position information indicating a position of the reflection point on a contact surface where the contact portion contacts the living body, based on the position information, the orientation of the transducer, the path length of the reflected wave, a visual angle between the center axis of oscillation and the reflection point viewed from the oscillation side transducer, and the incident angle.

5. The ultrasonic oscillator according to claim 4, further comprising
a contact surface calculation unit that calculates contact surface shape information indicating a shape of the contact surface where the contact portion contacts the living body by using a plurality of said reflection point position information.

6. The ultrasonic oscillator according to claim 5, the controller further comprises a focus control unit that controls a focal position of ultrasonic waves oscillated from the plurality of transducers, by calculating time required for ultrasonic waves to reach the focal position from each transducer via the contact surface based on position information indicating the relative positional relationship between the plurality of transducers, orientations of the plurality of transducers, and the contact surface shape information, and adjusting phase of the ultrasonic waves by each of the plurality of transducers in accordance with a calculation result.

7. The ultrasonic oscillator according to any one of claims 2 to 6, further comprising
a driver that is connected to the retention portion and deforms the concave portion by driving,
wherein the controller further comprises a focus control unit that controls the driver so as to deform the concave portion and adjust oscillation directions of the ultrasonic waves oscillated from the plurality of transducers based on a result of comparing position information indicating the relative positional relationship between the plurality of transducers and orientations of the plurality of transducers with aiming position and aiming orientation of the plurality of transducers, thereby controlling a focal position of the ultrasonic waves oscillated from the plurality of transducers.

8. The ultrasonic oscillator according to any one of claims 1 to 7, wherein the distance calculation unit calculates the distance by multiplying a propagation speed by the time from oscillation to sensing.

9. The ultrasonic oscillator according to any one of claims 1 to 8, wherein the angle calculation unit calculates the visual angle by calculating an oscillation angle having a spectrum corresponding to the spectrum of the sensing signal based on the correspondence relationship between oscillation angle and spectrum held in advance.

10. The ultrasonic oscillator according to any one of claims 3 to 6, wherein the angle calculation unit calculates the incident angle by comparing the estimated intensity of sensing signal when the sensing side transducer senses the ultrasonic wave vertically and the intensity of actually received sensing signal.

11. The ultrasonic oscillator according to any one of claims 1 to 10, wherein the controller controls the phases of the ultrasonic waves oscillated from the plurality of transducers such that the ultrasonic waves oscillated from the plurality of transducers cancel each other in a partial area, thereby cancelling the heating in the partial area.

12. The ultrasonic oscillator according to any one of claims 1 to 11, further comprising one of liquid and gel filling the hollow of the retention portion.

13. The ultrasonic oscillator according to any one of claims 11 to 12, further comprising an image processing unit that generates and outputs image data on a reflecting target including the target using sensing signals based on reflected waves among the received sensing signals.

14. The ultrasonic oscillator according to claim 13, wherein
the image processing unit compares a position of the target with the focal position in the image data and generates and outputs instructing information for guiding the focal position to the position of the target.

15. The ultrasonic oscillator according to claim 13 or 14, wherein
the controller compares a position of the target with the focal position in the image data and stops, when deviation between the position of the target and the focal position exceeds a predetermined criterion, the oscillation of the ultrasonic waves by the plurality of transducers.

16. A method executed by an ultrasonic oscillator comprising: a plurality of transducers that oscillates and/or senses the ultrasonic waves; a retention portion that retains the plurality of transducers on a deformable concave portion forming a concave thereof opposing the heating target, so as to retain the plurality of transducers movable in conformity with the concave portion deforming in a state where the plurality of transducers is arranged along the concave; and a controller that controls the transducers so as to oscillate the ultrasonic waves, the method comprises:

   receiving sensing signals output from the transducers which sensed the ultrasonic waves oscillated from any of the plurality of transducers,
   specifying a sensing signal related to a direct wave included in the received sensing signals,
   calculating the distance between the oscillation side transducer and the sensing side transducer based on time from oscillation to sensing of the direct wave,
   calculating a visual angle between a center axis of oscillation and the sensing side transducer viewed from the oscillation side transducer, based on the sensing signal related to the direct wave, and
   correcting position information indicating a relative positional relationship between the plurality of transducers by using the distance and the visual angle.

17. A program executed by a computer of an ultrasonic oscillator comprising: a plurality of transducers that oscillates and/or senses the ultrasonic waves; and a retention portion that retains the plurality of transducers on a deformable concave portion forming a concave thereof opposing the heating target, so as to retain the plurality of transducers movable in conformity with the concave portion deforming in a state where the plurality of transducers is arranged along the concave, wherein the computer controls the transducers so as to oscillate the ultrasonic waves, the program causes the computer to execute:

   receiving sensing signals output from the transducers which sensed the ultrasonic waves oscillated from any of the plurality of transducers,
   specifying a sensing signal related to a direct wave included in the received sensing signals,
   calculating the distance between the oscillation side transducer and the sensing side transducer based on time from oscillation to sensing of the direct wave,
   calculating a visual angle between a center axis of oscillation and the sensing side transducer viewed from the oscillation side transducer, based on the sensing signal related to the direct wave, and
   correcting position information indicating a relative positional relationship between the plurality of transducers by using the distance and the visual angle.

## FIG. 1

# FIG. 2

# FIG. 3

```
                              ┌─10
┌──────────────────────────────────────┐
│  ┌─────────────────┐ ┌─21             │
│──│    RECEIVER      │                 │
│  └─────────────────┘                  │
│  ┌──────────────────────────┐ ┌─22    │
│──│ DIRECT WAVE SPECIFYING UNIT │      │
│  └──────────────────────────┘         │
│  ┌────────────────────────────┐ ┌─23  │
│──│ REFLECTED WAVE SPECIFYING UNIT │   │
│  └────────────────────────────┘       │
│  ┌──────────────────────────┐ ┌─24    │
│──│ DISTANCE CALCULATION UNIT │        │
│  └──────────────────────────┘         │
│  ┌────────────────────────┐ ┌─25      │
│──│  ANGLE CALCULATION UNIT │          │
│  └────────────────────────┘           │
│  ┌──────────────────┐ ┌─26            │
│──│  CORRECTION UNIT  │                │
│  └──────────────────┘                 │
│  ┌──────────────────────────┐ ┌─27    │
│──│ POSTURE CALCULATION UNIT  │        │
│  └──────────────────────────┘         │
│  ┌────────────────────────────────┐ ┌─28 │
│──│ REFLECTION POINT CALCULATION UNIT │  │
│  └────────────────────────────────┘   │
│  ┌────────────────────────────────┐ ┌─29 │
│──│ CONTACT SURFACE CALCULATION UNIT │   │
│  └────────────────────────────────┘   │
│  ┌──────────────────────┐ ┌─30        │
│──│  FOCUS CONTROL UNIT   │            │
│  └──────────────────────┘             │
│  ┌──────────────────────┐ ┌─31        │
│──│ IMAGE PROCESSING UNIT │            │
│  └──────────────────────┘             │
└──────────────────────────────────────┘
```

# FIG. 4

START

INITIALIZATION/SELF-DIAGNOSIS — S101

ABNORMALITY DETECTED? — S102

YES → DEGENERATE OPERATING / POWER OFF — S103

NO

HEATING STOP INSTRUCTION? — S104

YES → TO "SCAN ONLY MODE" — S105

NO

HEATING INSTRUCTION? — S106

YES → TO "SCAN & HEAT MODE" — S107

NO

CURVATURE CHANGE INSTRUCTION? — S108

YES → CHANGE CURVATURE OF MEMBRANE — S109

NO

PROCESS OF DETERMINING RELATIVE POSITIONS/POSTURES OF TRANSDUCERS AND MEMBRANE (CALIBRATION PROCESS) — S110

SCANNING/IMAGING PROCESS — S111

SCAN & HEAT MODE? — S112

YES → HEATING PROCESS — S113

NO

# FIG. 5

PROCESS OF DETERMINING
RELATIVE POSITIONS/POSTURES
OF TRANSDUCERS AND MEMBRANE
(CALIBRATION PROCESS)

S201

OSCILLATE PULSE FROM TRANSDUCER 1
SENSE BY OTHER TRANSDUCERS AND RECORD RECEIVED DATA
OSCILLATE PULSE FROM TRANSDUCER 2
SENSE BY OTHER TRANSDUCERS AND RECORD RECEIVED DATA

.
.
.

OSCILLATE PULSE FROM TRANSDUCER N
SENSE BY OTHER TRANSDUCERS AND RECORD RECEIVED DATA

S202

RELATIVE POSITION/POSTURE DETERMINATION PROCESS
(DETERMINE POSITION AND ORIENTATION OF EACH TRANSDUCER
BASED ON RECEIVED DATA GROUP)

S203

MEMBRANE SHAPE DETERMINATION PROCESS
(CALCULATE SHAPE OF REFLECTION SURFACE
(CONTACT SURFACE) BASED ON RECEIVED DATA GROUP,
POSITION AND ORIENTATION OF EACH TRANSDUCER)

END

# FIG. 6

RELATIVE POSITION/POSTURE DETERMINATION PROCESS

S301
CALCULATE $\tau_{ij}$ FROM WAVEFORM OF DIRECT WAVE $T_i \to T_j$, AND CALCULATE DISTANCE $|P_i - P_j|$ BETWEEN $T_i - T_j$

S302
CUT OUT DIRECT WAVE FROM RECEIVED WAVEFORM OF $T_i \to T_j$, CALCULATE SPECTRUM USING FFT ETC., AND CALCULATE ANGLE $\theta_{i \to j}$

S303
PROCESS COMPLETE FOR ALL COMBINATIONS OF $i$, $j$?

NO

YES

S304
DETERMINE $P_i$ ($i = 1...N$) USING LEAST-SQUARE METHOD ETC.

S305
SELECT 2 OR MORE COMBINATIONS AS TO $j$ FROM COMBINATIONS OF $P_i$, $P_j$, $\theta_{i \to j}$ AND OBTAIN $d_i$

S306
PROCESS COMPLETE FOR ALL $i$?

NO

YES

END

24

# FIG. 7

EP 3 446 652 A1

## FIG. 8

MEMBRANCE SHAPE DETERMINATION PROCESS

S401
CALCULATE REFLECTED WAVE ARRIVAL TIME $\tau'_{ij}$
FROM RECEIVED WAVEFORM OF $T_i \rightarrow T_j$, AND
CALCULATE LENGTH OF $\text{path}(T_i \rightarrow R_{ij} \rightarrow T_j)$

S402
CUT OUT RECEIVED WAVEFORM OF REFLECTED WAVE OF $T_i \rightarrow T_j$,
CALCULATE SPECTRUM BY FFT ETC.,
CALCULATE ANGLE $\phi_{i \rightarrow R_{ij}}$

S403
CALCULATE ANGLE $\theta_{R_{ij} \rightarrow j}$ FROM GAIN OF REFLECTED WAVE AT $T_j$

S404
PROCESS COMPLETE FOR
ALL COMBINATIONS OF i, j?

NO

YES

S405
CALCULATE $R_{ij}$ USING LEAST-SQUARE METHOD ETC.
FROM $\{P_i, d_i\}$, LENGTH OF $\text{path}(T_i \rightarrow R_{ij} \rightarrow T_j)$,
ANGLE$\{\phi_{i \rightarrow R_{ij}}, \theta_{R_{ij} \rightarrow j}\}$

S406
CALCULATE CURVED SURFACE SHAPE NEAR
REFLECTION POINT BY SPLINE INTERPOLATION, AND
DETERMINE SHAPE OF CONTACT SURRACE TT

END

## FIG. 9

# FIG. 10

```
        ( SCANNING/IMAGING PROCESS )
                      │
                      ▼                              S501
        ┌─────────────────────────────────────┐
        │   DETERMINE CONVERGENCE DIRECTION OF │
        │    ULTRASONIC BEAM FOR IMAGING, AND  │
        │      DETERMINE FOCAL POINT SO THAT   │
        │  BEAM CONVERGES IN DETERMINED DIRECTION │
        └─────────────────────────────────────┘
                      │
                      ▼                              S502
        ┌─────────────────────────────────────┐
        │  SETTING FOR FOCUSING BY PHASE CONTROL │
        └─────────────────────────────────────┘
                      │
                      ▼                              S503
        ┌─────────────────────────────────────┐
        │      OSCILLATE SCANNING PULSE FROM   │
        │     EACH TRANSDUCER BY PHASE CONTROL │
        │       IN ACCORDANCE WITH SETTING, AND │
        │  RECEIVE REFLECTED WAVES BY EACH TRANSDUCER │
        └─────────────────────────────────────┘
                      │
                      ▼                              S504
                    STRUCTURE DATA
      NO          NECESSARY FOR IMAGING IS
                      COMPLETED?
                      │ YES                          S505
                      ▼
        ┌─────────────────────────────────────┐
        │           IMAGE PROCESSING          │
        │    GENERATE CROSS-SECTIONAL IMAGE   │
        │    GENERATE BLOOD FLOW INFORMATION  │
        │            TARGET CAPTURE           │
        │            REFLESH DISPLAY          │
        └─────────────────────────────────────┘
                      │
                      ▼
                   ( END )
```

# FIG. 11

# FIG. 12

```
           ┌──────────────────────┐
           │   HEATING PROCESS     │
           └──────────────────────┘
                      │
                      ▼
              ╱────────────────╲  S601
        ╱─────────────────────────────╲
   NO  ╱    HEATING INCOMPLETE          ╲
 ◄─────        AREA EXISTS?
        ╲                               ╱
         ╲─────────────────────────────╱
                      │ YES    S602
                      ▼
           ┌──────────────────────────┐
           │  SELECT HEATING TARGET   │
           │ AREA FROM HEATING        │
           │ INCOMPLETE AREA          │
           └──────────────────────────┘
                      │          S603
                      ▼
           ┌──────────────────────────────────┐
           │ CALCULATE PHASE DIFFERENCE OR     │
           │ WAVEFORM BETWEEN TRANSDUCERS FOR  │
           │ FOCUSING AT HEATING TARGET        │
           └──────────────────────────────────┘
                      │
                      ▼
              ╱────────────────╲  S604
        ╱─────────────────────────────╲
       ╱   HEATING TARGET AREA IS       ╲   NO
       ╲ WITHIN THE RANGE FOCUS          ╱──────┐
        ╲ CONTROLABLE BY PHASE CONTROL? ╱       │
         ╲─────────────────────────────╱        │
                      │ YES                      ▼  S607
                      │              ┌──────────────────────────┐
                      │              │ INSTRUCT CURVATURE        │
                      │              │ CHANGE, AND GENERATE      │
                      │              │ DEVICE MOVEMENT           │
                      │              │ NAVIGATION                │
          S605        │              └──────────────────────────┘
           ▼          │                          │
┌──────────────────────────┐                     ▼
│ OSCILLATE ULTRASONIC WAVE │              ╱──────────────╲  S608
│ FOR HEATING FROM PLURAL   │         ╱─────────────────────╲
│ TRANSDUCERS               │        ╱   DEVIATION BETWEEN    ╲  NO
└──────────────────────────┘        ╲  HEATING POSITION AND   ╱───┐
           │  S606                    ╲ HEATING TARGET EXCEEDS╱    │
           ▼                           ╲    CRITERION?       ╱     │
┌──────────────────────────┐            ╲─────────────────╱       │
│         UPDATE            │                  │ YES               │
│ CUMULATIVE HEATING AMOUNT │                  ▼       S609        │
│ OF HEATED PORTION, AND    │       ┌──────────────────────────┐  │
│ CONFIRM COMPLETION OF     │       │ ISSUE HEATING STOP        │  │
│ HEATING                   │       │ INSTRUCTION               │  │
└──────────────────────────┘       └──────────────────────────┘  │
           │                                  │                   │
           └──────────────────┬───────────────┴───────────────────┘
                              │
                              ▼
                        ┌──────────┐
                        │   END    │
                        └──────────┘
```

## INTERNATIONAL SEARCH REPORT

International application No.

PCT/JP2016/053803

A. CLASSIFICATION OF SUBJECT MATTER
*A61B18/00*(2006.01)i

According to International Patent Classification (IPC) or to both national classification and IPC

B. FIELDS SEARCHED

Minimum documentation searched (classification system followed by classification symbols)
A61B17/22, A61B17/225, A61B18/00, A61N7/00-7/02

Documentation searched other than minimum documentation to the extent that such documents are included in the fields searched
Jitsuyo Shinan Koho        1922-1996   Jitsuyo Shinan Toroku Koho   1996-2016
Kokai Jitsuyo Shinan Koho  1971-2016   Toroku Jitsuyo Shinan Koho   1994-2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | Microfilm of the specification and drawings annexed to the request of Japanese Utility Model Application No. 12972/1989(Laid-open No. 28212/1990) (Siemens AG.), 23 February 1990 (23.02.1990), specification, page 17, line 18 to page 18, line 1; page 20, line 14 to page 21, line 15; page 24, line 17 to page 26, line 16; fig. 3 & JP 2-28212 U        & EP 0327917 A1 & EP 0327917 B1        & US 4957099 A column 5, lines 34 to 36; column 6, lines 32 to 51; column 7, line 51 to column 8, line 32; fig. 3 | 1-17 |

[X] Further documents are listed in the continuation of Box C.        [ ] See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 05 April 2016 (05.04.16) | 26 April 2016 (26.04.16) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2016/053803

C (Continuation). DOCUMENTS CONSIDERED TO BE RELEVANT

| Category* | Citation of document, with indication, where appropriate, of the relevant passages | Relevant to claim No. |
|---|---|---|
| A | JP 2003-517856 A (Transurgical Inc.), 03 June 2003 (03.06.2003), paragraphs [0014], [0016]; fig. 1 & JP 3848572 B2 & AU 7362400 A & EP 1229820 A2 & EP 1229820 A4 & US 6599256 B1 column 5, lines 37 to 67; column 6, lines 30 to 43; fig. 1 & WO 2001/017455 A2 & WO 2001/017455 A3 | 1-17 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004066856 A
- JP H8131454 B
- JP 2015056750 W